# FASCICULE DE BREVET EUROPEEN

(11) **EP 3 801 778 B1**
(45) Date de publication et mention de la délivrance du brevet: **10.01.2024**
(21) Numéro de dépôt: 19735371.7
(22) Date de dépôt: 06.06.2019
(51) Int. Cl.: A61Q 19/00, A61Q 5/00, A61Q 5/08, A61K 8/9789, A61K 36/185, A61P 17/00, A61P 17/08

(54) **UTILISATION D'UN EXTRAIT DE BIXA ORELLANA**
VERWENDUNG EINES BIXA-ORELLANA-EXTRAKTES
USE OF A BIXA ORELLANA EXTRACT

(30) Priorité: 11.06.2018 FR 1855079
(43) Date de publication de la demande: 14.04.2021
(73) Titulaire: BASF Beauty Care Solutions France SAS, 69007 Lyon (FR)
(72) Inventeur: BERTHELEMY, Nicolas, 54530 PAGNY SUR MOSELLE (FR); DANOUX, Louis, 54420 SAULXURES-LES-NANCY (FR); MOSER, Philippe, 54130 DOMMARTEMONT (FR); PAIN, Sabine, 38200 VIENNE (FR); PELLETIER, Nicolas, 69004 LYON (FR)
(74) Mandataire: Cabinet Beau de Loménie
(86) Numéro de dépôt international: PCT/FR2019/051350
(87) Numéro de publication internationale: WO 2019/239035

(56) Documents cités:
- EP-A1- 2 441 435
- FR-A1- 2 875 701
- FR-A1- 2 939 039
- JP-A- H08 231 351
- US-A- 6 043 202
- DATABASE WPI Week 200672 Thomson Scientific, London, GB; AN 2006-693079 XP002789089, -& JP 2006 257058 A (KOEI KOGYO KK) 28 septembre 2006 (2006-09-28)

## Description

La présente invention concerne l'utilisation d'un extrait de *Bixa orellana* pour prévenir et/ou réduire la sécrétion de sébum et/ou les manifestations inesthétiques, désagréables et/ou inconfortables liées à la sécrétion de sébum notamment dans des compositions cosmétiques ou pharmaceutiques, notamment dermatologiques.

Le sébum est produit par les glandes sébacées de la peau et est constitué d'un mélange complexe de lipides notamment des triglycérides (30-50 %), des acides gras libres (15-30 %), des cire esters (26-30 %) et du squalene (12-20 %).

Les glandes sébacées sont localisées dans la peau où, en association avec le follicule pileux, elles forment l'unité pilosebacée. Elles sont plus nombreuses sur le cuir chevelu et le visage atteignant jusqu'à 400-900 glandes / cm ². La fonction principale des glandes sébacées est la sécrétion de sébum à partir des sébocytes matures. La sécrétion est de type holocrine c'est à dire qu'en excrétant le sébum dans le canal folliculaire, les sébocytes meurent. Les sébocytes périphériques prolifèrent et se différencient ensuite en sébocytes matures au centre de la glande, ces derniers se caractérisant par une haute densité de gouttelettes lipidiques cytoplasmiques. Le renouvellement complet de sébocytes dans les glandes sébacées dure environ 4 semaines .

La production de sébum est indispensable pour un bon état et fonctionnement de la peau. Le sébum est destiné notamment à protéger la peau du dessèchement, des microbes par acidification, et préserver sa souplesse. Des rôles antibactériens et de transport de phéromones lui ont été attribués, ainsi qu'un rôle antioxydant notamment lié à la production de vitamine E.

Un niveau trop faible de production de sébum conduit ainsi à l'apparition d'une peau sèche et plus fragile et à la perte de la fonction du follicule pileux pouvant se traduire par la chute de cheveux.

Si la sécrétion de sébum est utile par ces fonctions protectrices de la peau, celle-ci confère à la peau lorsqu'elle est excessive, des imperfections comme par exemple un aspect souvent qualifié de luisant, épais, gras, une dilatation des pores et/ou la formation de points noirs, une hyperkératinisation de la peau, autant de manifestations souvent perçues comme inesthétiques et/ou désagréables et/ou inconfortables. Ces manifestations caractérisent une peau dite « grasse ».

La quantité et la qualité de sébum sécrétée varie selon le type de peaux par exemple caucasiennes, asiatiques et africaines, toutefois le phénomène de peau grasse concernerait 80% des personnes à l'échelle mondiale et constitue donc un véritable problème cosmétique multiethnique.

En outre, cette séborrhée peut être à l'origine de réelles pathologies telles que l'eczéma séborrhéique, et/ou l'hypersécrétion du nourrisson (communément désigné sous le terme « croutes de lait »).

Si l'origine principale de la peau grasse est une hyperactivité des sébocytes, avec une sécrétion et une excrétion excessive de sébum par les glandes sébacées, de multiples facteurs intrinsèques (âge, hormones, stress) et/ou extrinsèques (température, humidité, agents agressifs tels que polluants) influencent la qualité et/ou la quantité de sébum sécrété. Ainsi, les hormones androgènes et l'excès de facteurs de croissance produits pendant la puberté sont les facteurs principaux impliqués dans le développement des peaux grasses. Par ailleurs, un excès du facteur de croissance Insulin Growth factor de type 1 (IGF1) est associé d'une part à une hyperprolifération des sébocytes conduisant à une surproduction de sébum et d'autre part à une hyperprolifération et différentiation des kératinocytes conduisant à une hyperkératinisation et donc une peau épaissie.

De nombreux actifs existent dans les domaines cosmétiques et dermatologiques pour prévenir et/ou diminuer la sécrétion de sébum de manière directe. Ces agents dits séborégulateurs réduisent directement la production de sébum par les glandes sébacées comme par exemple le gluconate de zinc et l'acide azélaique, la sarcosine et/ou un extrait d'*Orthosiphon stamineus.* D'autres agents sont aussi utilisés pour leur action indirecte sur la sécrétion de sébum, en particulier les agents exfoliants, stimulateurs du renouvellement cellulaire, astringent et/ou matifiant par absorption du sébum.

Il existe toutefois un besoin constant de nouvel actif capable de diminuer la sécrétion de sébum, qui soit à la fois visible et durable, sans engendrer d'effet rebond, ni irritation de la peau et sans perte de son éclat, et capable de préserver l'hydratation de la peau. Il existe également un besoin de nouvel actif capable en outre de prévenir et/ou diminuer les manifestations inesthétiques et/ou désagréables et/ou inconfortables de la sécrétion de sébum telles que l'aspect luisant, gras de la peau et/ou des cheveux, la visibilité des pores et/ou la formation de points noirs et/ou l'hyperkératinisation de de la peau notamment son aspect épais.
La présente invention vise ainsi à résoudre ces besoins par un nouvel agent inhibiteur de la sécrétion de sébum, utile comme alternatif aux actifs disponibles sur le marché.

Selon la présente invention, il a en effet été découvert de manière particulièrement surprenante et inattendue que l'extrait de *Bixa orellana* permet de résoudre l'ensemble de ces problèmes techniques. L'extrait de *Bixa orellana* selon l'invention permet en effet de prévenir et/ou réduire la sécrétion de sébum, en particulier des lipides totaux et/ou neutres par les sébocytes de manière très efficace de la peau incluant du cuir chevelu notamment en inhibant l'activité de IGF-1 et/ou en stimulant la synthèse de la protéine IGBP3 (IGF1 binding protein 3), inhibiteur d'activité de IGF-1. L'extrait selon l'invention permet ainsi de diminuer la prolifération des sébocytes. Cet effet est à la fois visible et durable, sans engendrer d'effet rebond. L'extrait selon l'invention présente également l'avantage de préserver l'hydratation et l'éclat de la peau.

En inhibant l'activité d'IGF1 directement et/ou via la stimulation de la synthèse de la protéine IGBP3 (IGF1 binding protein 3), l'extrait de *Bixa orellana* permet de prévenir et/ou diminuer les manifestations inesthétiques et/ou désagréables et/ou inconfortables de la sécrétion de sébum telles que l'aspect luisant et/ou gras de la peau et/ou de cheveux. En limitant la prolifération et la différentiation des kératinocytes, l'extrait selon l'invention permet de diminuer l'hyperkératinisation de la peau et ainsi l'aspect épais de la peau. L'extrait selon l'invention présente l'avantage de stimuler la synthèse et/ou l'activité de collagène de type IV par les kératinocytes ce qui permet de resserrer les pores de la peau et/ou de diminuer le nombre de pores dilatés et/ou de prévenir la dilatation des pores de la peau et/ou la visibilité des pores de la peau et/ou de réduire la formation de points noirs. De manière générale, le grain de peau est ainsi affiné.

Par ailleurs, l'extrait selon l'invention permet de diminuer l'activité sécrétrice des sébocytes sans la stopper complètement, maintenant ainsi un niveau minimum de sécrétion de sébum, indispensable à l'homéostasie de la peau et/ou des cheveux. A long terme, l'extrait selon l'invention ne dessèche ainsi pas la peau.

Par son action complète et ces activités complémentaires et dénuée des inconvénients de certains produits du marché cosmétique, l'extrait selon l'invention est donc un excellent agent inhibiteur de la sécrétion de sébum et de ses manifestations inesthétiques et/ou désagréables et/ou inconfortables. Il est ainsi particulièrement utile pour le traitement et/ou soin cosmétique des peaux normales, grasses, très grasses et/ou mixtes et/ou cheveux normaux et/ou gras.

De même, il est ainsi particulièrement utile pour le traitement et /ou soin cosmétique des peaux caucasiennes, asiatiques et/ou africaines et/ou des cheveux caucasiens, asiatiques et/ou africains.

*Bixa orellana* est un arbuste qui appartient au genre Bixa, à la famille des Bixaceae. Elle est communément dénommée « roucou » ou arbre à rouge à lèvres.

Des extraits de cet arbuste ont déjà été décrits dans des applications cosmétiques pour un effet blanchissant (JP08012563). Des extraits huileux de graines ont également été décrits comme agent photoprotecteur (FR2555447) et comme agent hydratant (FR2798591).

Aucune des propriétés connues de cet extrait ne laissait néanmoins présager de son effet pour prévenir et/ou limiter la sécrétion de sébum et/ou les manifestations inesthétiques de la sécrétion de sébum comme découvert dans la présente invention.

L'extrait de *Bixa orellana* présente l'avantage d'agir sur les sébocytes de manière directe et avec un effet durable, à long terme. En outre, l'extrait selon l'invention prévient et/ou réduit les imperfections cutanées liées à une production importante de sébum. Il a notamment été observé que l'extrait selon l'invention affine le grain de la peau en diminuant la visibilité des pores, notamment réduisant la taille des pores et en le rendant plus régulier, plus lisse.

Certaines de ces propriétés de l'extrait de *Bixa orellana* selon l'invention seront notamment démontrées plus en détails dans les exemples fournis ci-après.

La présente invention a ainsi pour objet l'utilisation cosmétique non thérapeutique d'un extrait hydrosoluble de *Bixa orellana* pour prévenir et/ou réduire la sécrétion de sébum et/ou pour prévenir et/ou réduire les manifestations inesthétiques et/ou désagréables et/ou inconfortables liées à la sécrétion de sébum, notamment pour prévenir et/ou réduire la visibilité des pores de la peau et/ou la formation de points noirs, et/ou l'aspect luisant et/ou gras de la peau et/ou des cheveux et/ou l'aspect épais de la peau, l'extrait de *Bixa orellana* étant un extrait de graine obtenu par extraction aqueuse.

La présente invention a également pour objet un extrait hydrosoluble de *Bixa orellana* pour son utilisation seul ou dans une composition pharmaceutique, notamment dermatologique dans le traitement et/ou la prévention d'au moins une pathologie associée à une hyperproduction de sébum, en particulier les hyperséborrhées pathologiques, l'eczéma séborrhéique et/ou l'hypersécrétion du nourrisson et/ou l'une quelconque de leurs combinaisons, l'extrait de *Bixa orellana* étant un extrait de graine obtenu par extraction aqueuse.

La présente invention a également pour objet l'utilisation selon l'invention d'une composition cosmétique ou pharmaceutique, notamment dermatologique comprenant un extrait hydrosoluble *de Bixa orellana,* l'extrait de *Bixa orellana* étant un extrait de graine obtenu par extraction aqueuse, en association avec au moins un agent choisi parmi les agents séborégulateurs, préférentiellement choisi parmi la sarcosine, le salicylate de zinc, le gluconate de zinc, l'acide azélaique, un extrait d'Orthosiphon stamineus et/ou leurs dérivés, et/ou au moins un agent à propriété complémentaire choisi parmi les agents exfoliants, hydratants, kératolytiques, stimulant la synthèse de fibronectine, de protection du facteur de croissance des fibroblastes (FGF2), de stimulation de croissance des fibroblastes, agent antibactérien, absorbeur de sébum, comédolytique, antibiotique local et l'un quelconque de leur mélanges.

La présente invention a également pour objet l'utilisation cosmétique non thérapeutique d'une telle composition cosmétique pour prévenir et/ou réduire la sécrétion de sébum et/ou pour prévenir et/ou réduire les manifestations inesthétiques et/ou désagréables et/ou inconfortables liées à la sécrétion de sébum, notamment pour prévenir et/ou réduire la visibilité des pores de la peau et/ou la formation de points noirs et/ou l'aspect luisant et/ou gras de la peau et/ou des cheveux et/ou l'aspect épais de la peau..

La présente description décrit un procédé de soin et/ou traitement cosmétique (qui ne fait pas partie de l'invention) comprenant l'application par voie topique, en particulier quotidienne, sur au moins une zone de peau, avantageusement du cuir chevelu, d'un extrait *de Bixa orellana* selon l'invention ou d'une composition cosmétique selon l'invention pour prévenir et/ou réduire la sécrétion de sébum et/ou pour prévenir et/ou réduire les manifestations inesthétiques et/ou désagréables et/ou inconfortables liées à la sécrétion de sébum, notamment pour prévenir et/ou réduire la visibilité des pores de la peau et/ou la formation de points noirs, et/ou l'aspect luisant et/ou gras de la peau et/ou des cheveux et/ou l'aspect épais de la peau.

La présente invention a pour objet l'utilisation cosmétique non thérapeutique d'un extrait hydrosoluble *de Bixa orellana* pour prévenir et/ou réduire la sécrétion de sébum, préférentiellement sous la forme d'une composition cosmétique, l'extrait de *Bixa orellana* étant un extrait de graine obtenu par extraction aqueuse.

La présente invention a ainsi pour objet l'utilisation cosmétique non thérapeutique d'un extrait hydrosoluble de *Bixa orellana* pour prévenir et/ou réduire la sécrétion de sébum et/ou les manifestations inesthétiques et/ou désagréables et/ou inconfortables liées à la sécrétion de sébum, l'extrait de *Bixa orellana* étant un extrait de graine obtenu par extraction aqueuse.

On entend par « prévenir et/ou réduire la sécrétion de sébum » diminuer ou empêcher l'augmentation de la quantité de sébum sécrétée par les sébocytes dans la peau incluant le cuir chevelu, et avantageusement les lipides totaux et/ou neutres du sébum, notamment le squalène et/ou diminuer ou empêcher la prolifération des sébocytes dans la peau, incluant le cuir chevelu.

La quantité de sébum sécrétée par les sébocytes peut être mesurée in vitro par dosage de la quantité de lipides cellulaires produits dans les sébocytes en culture avec une coloration au Nile Red selon la méthode décrite Greenspan P et al. (Nile red: a sélective fluorescent stain for intracellular lipid droplets. J Cell Biol. 100(3):965-73, (1985)) tel que décrit dans l'exemple 2.

La quantité de sébum sécrétée peut également être mesurée in vivo par l'analyse clinique et instrumentale de prélèvements des sécrétions de sébum sur des prélèvements tels que les patchs Sébutape^{™} selon la méthode classique et/ou par application d'un sébumètre sur la zone de peau concernée, préférentiellement l'appareil Sebumeter^{™} commercialisé par la société Courage + Khazaka Electronic GmbH, encore préférentiellement le modèle SM815.

Avantageusement l'extrait selon l'invention permet de diminuer d'au moins 5%, préférentiellement au moins 10%, encore préférentiellement 20% la quantité de sébum sécrété par rapport au contrôle en l'absence d'extrait comme cela est démontré dans l'exemple 2.

La prolifération des sébocytes dans la peau peut être évaluée par dénombrement des sébocytes en présence de l'extrait selon l'invention par le maculage d'ADN avec le réactif de Hoechst selon la méthode décrite par Daxhelet et al (Spectrofluorometry of dyes with DNAs of different base composition and conformation; Analytical Biochemistry Volume 179, Issue 2, June 1989, Pages 401-403 Analytical Biochemistry 179:401-403 (1989) et présentée en exemple 2.

Avantageusement l'extrait selon l'invention permet de diminuer d'au moins 5%, préférentiellement au moins 10%, encore préférentiellement au moins 15% la prolifération des sébocytes par rapport au contrôle en l'absence d'extrait préférentiellement comme cela est démontré dans l'exemple 2.

On entend par « manifestations inesthétiques et/ou désagréables et/ou inconfortables liées à la sécrétion de sébum » l'aspect luisant et/ou gras de la peau et/ou des cheveux, la visibilité des pores de la peau et/ou la formation de points noirs, et/ou l'aspect épais de la peau. Selon un mode particulier de l'invention, les manifestations inesthétiques et/ou désagréables et/ou inconfortables de la sécrétion de sébum sont choisies parmi l'aspect luisant et/ou gras de la peau et/ou des cheveux, la formation de points noirs, l'aspect épais de la peau.

On entend au sens la présente invention par « diminuer la visibilité des pores cutanés » resserrer les pores cutanés, c'est à dire diminuer le diamètre d'ouverture des pores, et/ou la densité et/ou l'aire des pores à la surface de la peau, et/ou prévenir la dilatation des pores cutanés. L'extrait selon l'invention permet donc pour diminuer l'ouverture et/ou la densité des pores à la surface de la peau.

La visibilité des pores de la peau peut être mise en évidence in vivo par une évaluation dite « scoring » par un dermatologique sur une zone prédéfinie après application d'une composition comprenant l'extrait selon l'invention, notamment comme décrit dans l'exemple 5. Elle peut également être mise en évidence par une méthode instrumentale objective par une analyse d'image permet d'extraire et de quantifier des paramètres spécifiques des photographies hautes résolution en configuration polarisée croisée du visage des volontaires avant et après application d'une composition comprenant l'extrait selon l'invention.

La densité des pores cutanés peut également être mesurée *in vivo* par imagerie notamment par la technique de projection de franges, en mesurant le paramètre dit de curvature.

Dans un mode de réalisation préféré de l'invention, l'extrait de *B. orellana* selon l'invention est en quantité efficace pour diminuer la visibilité des pores de la peau d'au moins 10%, préférentiellement d'au moins 20%, après 28 jours d'application d'une crème comprenant l'extrait de *B. orellana* selon l'invention, préférentiellement de graines, encore préférentiellement préparé dans les conditions décrites dans l'exemple 1a), préférentiellement formulé sous forme de composition cosmétique telle que celle décrite dans l'exemple 5.

On entend par « aspect épais de la peau » selon l'invention une hyperkératinisation de la peau. La diminution de l'hyperkératinisation de la peau peut être mesurée par mesure de la quantité de protéine IGF1-BP3 en présence de l'extrait selon l'invention sur des kératinocytes humains normaux en culture, notamment tel que présenté dans l'exemple 3. De manière avantageuse, l'extrait selon l'invention est en quantité efficace pour augmenter d'au moins 5%, préférentiellement au moins 10%, encore préférentiellement au moins 20% la quantité de protéine IGF1-BP3 synthétisée en présence de l'extrait selon l'invention.

Par peaux très grasses selon l'invention, on entend une peau qui a un indice lipidique mesuré par un sébumètre appliqué directement la zone de peau à évaluer supérieur ou égal à 150 microgrammes de sébum par cm².

Par peau normale selon l'invention, on entend une peau qui a un indice lipidique mesuré par un sébumètre appliqué directement la zone de peau à évaluer strictement inférieur à 120 microgrammes de sébum par cm², et préférentiellement supérieur ou égale à 100 microgrammes de sébum par cm².

Par peau grasse ou séborrhéique selon l'invention, on entend une peau qui a un indice lipidique mesuré par un sébumètre appliqué directement la zone de peau à évaluer allant de 120 et 149 microgrammes de sébum par cm².

Par peau mixte, on entend une peau qui présente plusieurs profils : grasse, très grasse, et/ou normale en fonction des zones du visage considéré, notamment une peau grasse sur la zone T et une peau normale à sèche sur le reste du visage.

Par cheveux normaux selon l'invention, on entend un cuir chevelu qui a un indice lipidique mesuré par un sébumètre appliqué directement la zone du cuir chevelu à évaluer inférieur ou égale à 100 microgrammes de sébum par cm².

Par cheveux gras selon l'invention, on entend un cuir chevelu qui a un indice lipidique mesuré à mesuré par un sébumètre appliqué directement la zone du cuir chevelu à évaluer strictement supérieur à 100 microgrammes de sébum par cm².

De manière préférentielle, on entend par appareil sébumètre, l'appareil Sebumeter^{™} commercialisé par la société Courage + Khazaka Electronic GmbH, encore préférentiellement le modèle SM815.

Au sens de la présente invention, on entend par « utilisation cosmétique » une utilisation non thérapeutique, non pharmaceutique de l'extrait selon l'invention, préférentiellement sur une peau saine, notamment un cuir chevelu sain, et/ou des cheveux sains, en particulier une utilisation destinée à tout ou partie de la peau, préférentiellement du cuir chevelu et/ou à une zone de peau, préférentiellement de cuir chevelu dite « saine », de façon particulière à une zone de peau « saine » et/ou une zone de cuir chevelu « saine ».

Au sens de la présente invention, on entend par partie de la peau préférentiellement de cuir chevelu et/ou zone de peau, préférentiellement de cuir chevelu dite « saine », une partie de la peau préférentiellement du cuir chevelu et/ou une zone de peau, préférentiellement du cuir chevelu qualifiée de non pathologique par un dermatologue, c'est-à-dire qui ne présente pas d'infection, d'inflammation, de cicatrice, de maladie ou d'affection cutanée telle que folliculite, candidose, psoriasis, ichtyose, pathologies liées à la mélanogénèse telle que le vitiligo, eczéma, acné, kératose actinique, carcinome, mélanome, furoncle ou dermatite notamment séborrhéique, alopécie ou de plaies ou de blessures. De façon particulière, on entend par partie de la peau, préférentiellement du cuir chevelu et/ou par zone de peau, préférentiellement du cuir chevelu dite « saine » une partie de la peau préférentiellement du cuir chevelu et/ou une zone de peau, préférentiellement du cuir chevelu qualifiée de « normale » par un médecin, particulièrement un dermatologue, c'est-à-dire, constituée de cellules « normales », c'est-à-dire non pathologiques, plus particulièrement non cancéreuses.

Ainsi, sauf indication contraire, on entend par peau « normale », cellules « normales », kératinocytes « normaux », sébocytes « normaux », des peaux ou cellules ne présentant pas de pathologie.

L'extrait selon l'invention est un extrait de *Bixa orellana.* L'extrait selon l'invention est extrait des graines de *Bixa orellana.*

L'extrait peut être obtenu par différentes méthodes d'extraction connues de l'homme du métier, choisis parmi la macération, la décoction à chaud, par broyage dont le broyage aux ultrasons, à l'aide d'un mixeur, ou encore l'extrait peut être obtenu par extraction dans l'eau en conditions subcritiques ou supercritiques (dioxyde de carbone). Préférentiellement, l'extraction est réalisée par macération.

L'extraction pourra être conduite à partir de matière sèche ou fraîche, avantageusement sèche, en quantité de 0,1 % à 20 % en poids, avantageusement de 1 % à 20%, très avantageusement de 5 % à 15 %, encore avantageusement de 10 % à 15 %, et encore plus avantageusement environ 10 % en poids par rapport au poids total de la matière et du solvant d'extraction.

L'extraction pourra être réalisée à une température allant de 4°C à 300°C, préférentiellement de 4°C à 100°C. Dans un mode préférentiel de réalisation de l'invention, l'extraction sera réalisée à une température allant de 60°C à 90°C, préférentiellement de 70°C à 85°C, encore préférentiellement à une température d'environ 80°C.

Selon un mode alternatif de réalisation, l'extraction sera réalisée à une température allant 4°C à 20°C, encore avantageusement à température ambiante, c'est-à-dire à environ 20°C.

Dans un autre mode alternatif de réalisation de l'invention, l'extraction sera réalisée dans l'eau en conditions subcritiques, à une température allant de 100°C à 300°C, avantageusement de 120°C à 250°C, encore avantageusement à 120°C. L'extraction peut être réalisée à une température donnée unique ou à des températures successives croissantes. Dans un mode de réalisation avantageux de l'invention, l'extraction sera réalisée à une température unique de 120°C. Dans un mode alternatif, elle sera conduite selon un gradient de trois températures croissantes comprises entre 100°C et 200°C, tel que 120°C, 140°C puis 160°C ou 110°C, 130°C puis 150°C, ou encore 120°C, 145°C puis 170°C.

On entend par extraction en « conditions subcritiques » une extraction en présence d'eau, dans des conditions de température supérieures à 100°C et de pression inférieure à 221 bars, telle que l'eau reste à l'état liquide mais possède une viscosité et une tension de surface inférieures à celle de l'eau à température ambiante, augmentant sa constante diélectrique.

Ainsi, la pression d'extraction sera comprise entre 150 bars et 250 bars, préférentiellement entre 200 et 221 bars, avantageusement dans un autoclave d'extraction sous pression.

De manière générale, l'extraction peut être conduite durant une période de 30 minutes à 24 heures, préférentiellement de 30 minutes à 12 heures, encore préférentiellement durant une période de 1 heure à 5 heures, et encore avantageusement durant une période de 1 heure à 2 heures. Très avantageusement, l'extraction sera conduite durant une période d'une heure.

L'extrait selon l'invention pourra être obtenu par extraction dans l'eau comme unique solvant.

L'extrait est obtenu par extraction aqueuse. Au sens de la présente invention, on entend par « extrait obtenu par extraction aqueuse » tout extrait obtenu par extraction avec une solution aqueuse contenant plus de 60% en poids, avantageusement au moins 70% en poids, en particulier au moins 80% en poids, plus particulièrement au moins 90% en poids, de façon particulière au moins 95% en poids, d'eau par rapport au poids total de la solution aqueuse, encore plus avantageusement ne contenant pas de glycol et de façon particulière ne contenant pas d'alcool, plus particulièrement ne contenant que de l'eau.

De manière avantageuse selon l'invention, l'extrait obtenu après l'étape d'extraction sera filtré à un seuil de coupure de 0,45 µm. Des étapes supplémentaires de décoloration et/ou désodorisation peuvent être effectuées sur l'extrait à n'importe quel stade de l'extraction et selon les techniques connues par l'Homme du métier. De façon particulière, l'extrait pourra être décoloré au charbon actif.

Par ailleurs, l'extrait obtenu après l'extraction peut être ensuite concentré par évaporation du solvant ou séché par exemple par lyophilisation ou par atomisation en présence d'un support d'atomisation comme par exemple la maltodextrine. L'extrait se présent alors sous forme de poudre. Selon un mode avantageux de l'invention, l'extrait de *Bixa orellana* obtenu, préférentiellement de graines sera atomisé en présence d'une concentration en poids de maltodextrine comprise entre 20% et 90%, préférentiellement entre 40 et 80%, encore préférentiellement environ 60% par rapport au poids total de la poudre obtenue.

L'extrait selon l'invention est hydrosoluble.

L'extrait selon l'invention est préférentiellement obtenu selon l'un des modes de réalisation décrit ci-dessous :
- dans un premier mode de réalisation de l'invention, l'extrait est obtenu par macération dans l'eau comme solvant d'une quantité de 10% en poids de graines broyées par rapport au poids du solvant et de la matière, durant une période d'une heure à une température de 80°C. L'extrait brut a été centrifugé, décanté puis filtré puis atomisé en présence de maltodextrine, en quantité finale de maltodextrine de 60% en poids par rapport au poids total de l'extrait final, dans les conditions décrites dans l'exemple 1a).
- Dans un second mode de réalisation de l'invention, l'extrait est obtenu par macération dans l'eau comme solvant d'une quantité de 10% en poids de graines broyées par rapport au poids total du solvant et de la matière, durant une période de 2 heures à une température de 20°C. L'extrait brut a été centrifugé, décanté puis filtré dans les conditions décrites dans l'exemple 1b).
- Dans un 3^{ème} mode de réalisation de l'invention, l'extrait est obtenu par macération d'une quantité de 20% de graines broyées dans l'eau comme solvant, durant une période de 2 heures à une température de 20°C. L'extrait est ensuite centrifugé, décanté, filtré dans les conditions décrites dans l'exemple 1c).
- Dans un 4^{ème} mode de réalisation, l'extrait est obtenu par macération d'une quantité de 10% en poids de graines broyées par rapport au poids total de la matière et du solvant, dans un mélange éthanol : eau (80 :20 ; v/v), à une température de 80°C durant une période de 1 heure. L'extrait est ensuite centrifugé, décanté, filtré puis atomisé en présence de maltodextrine, en quantité finale de maltodextrine de 80% en poids par rapport au poids total de l'extrait final, dans les conditions décrites dans l'exemple 1d).

Selon l'invention, l'extrait de *Bixa orellana* selon l'invention peut être utilisé seul sous forme d'ingrédient actif et/ou dans une composition cosmétique, préférentiellement destiné(e) à une application par voie topique.

Le terme "application par voie topique", utilisé ici, signifient appliquer l'extrait selon la présente invention éventuellement sous forme d'ingrédient actif et/ou de composition sur la surface de la peau incluant le cuir chevelu et/ou les cheveux notamment par application directe ou par vaporisation.

L'ingrédient actif et/ou les compositions cosmétiques contenant l'extrait selon l'invention sont en particulier destinées au soin et/ou au traitement cosmétique des peaux dites normales, grasses, très grasses et/ou mixtes et/ou cheveux dits normaux et/ou gras, en particulier des peaux grasses, très grasses et/ou mixtes et/ou cheveux gras.

Lorsqu'il est utilisé seul sous forme d'ingrédient actif, l'extrait selon l'invention est préférentiellement soluble et/ou dilué dans un solvant, notamment polaire, tel que l'eau, un alcool, un polyol, un glycol tel que le pentylène glycol et/ou le butylène glycol et/ou l'hexylène glycol et/ou le caprylyl glycol, ou un de leurs mélanges, préférentiellement un mélange hydroglycolique ou hydroalcoolique, encore préférentiellement contenant un glycol choisi parmi l'hexylène glycol, le caprylyl glycol et leurs mélanges.

Avantageusement, l'extrait selon l'invention est soluble et/ou solubilisé dans une solution aqueuse contenant du glycérol, avantageusement dans une solution contenant au moins 10 % en poids du glycérol, préférentiellement au moins 15 %, et encore avantageusement 17,5 % en poids de glycérol par rapport au poids total de la solution aqueuse.

Alternativement, l'extrait obtenu est dilué et/ou soluble dans une solution aqueuse contenant du caprylyl glycol, en particulier contenant entre 0,01 et 5 % en poids de caprylyl glycol, préférentiellement entre 0,1 et 1 % en poids de caprylyl glycol, par rapport au poids total la solution aqueuse.

Dans un autre mode de réalisation, l'extrait selon l'invention peut être incorporé dans une composition cosmétique comprenant au moins un excipient cosmétiquement acceptable.

On entend au sens de la présente invention par excipient « cosmétiquement acceptable » un composé et/ou solvant topiquement acceptable, c'est-à-dire n'induisant pas de réponse allergique au contact de la peau, incluant le cuir chevelu humain, non toxique, non instable, ou leurs équivalents, indue.

On entend au sens de la présente invention par « composition cosmétique » une composition non thérapeutique, c'est-à-dire une composition destinée à la prévention et/ou au soin de la peau, incluant le cuir chevelu, dite « normale » par un dermatologue, c'est-à-dire non pathologique. On entend ici par peau ou cuir chevelu « normal(e) » une peau ou un cuir chevelu sain(e) tel que défini précédemment. Dans un mode de réalisation préférentiel de l'invention, l'extrait selon l'invention est présent dans la composition cosmétique en une teneur comprise entre 1×10⁻⁴% à 10% en poids, préférentiellement de 1×10⁻⁴% à 5% en poids, encore avantageusement de 1×10⁻³% à 3% en poids, encore préférentiellement de 0,001 % et 0,1 % en poids, par rapport au poids total de la composition.

La composition cosmétique selon l'invention, peut se présenter sous toutes les formes galéniques classiquement utilisées pour une application topique sur la peau incluant le cuir chevelu, telles que les formes liquides ou solides ou même sous la forme de liquide sous pression. Elles peuvent notamment être formulées sous la forme d'une solution, aqueuse ou huileuse, une crème ou un gel aqueux ou un gel huileux, notamment en pot ou en tube, notamment un gel douche, un shampoing, un après-shampoing, un lait, une huile, une émulsion, un hydrogel, une microémulsion ou une nanoémulsion, notamment huile-dans-eau ou eau-dans-huile ou multiple ou siliconée, un sérum, une lotion, notamment en flacon de verre, de plastique ou en flacon doseur ou en aérosol ou en spray, une ampoule, un savon liquide ou solide, une pâte, une pommade, une mousse, un masque, une laque, un patch, un produit anhydre, de préférence liquide, pâteux ou solide, par exemple sous forme de bâtonnet notamment en stick ou en poudres. Il peut également s'agir d'un produit de maquillage ou d'un produit de démaquillage. En particulier, la composition cosmétique est choisie dans le groupe constitué par un sérum, une lotion, une crème, un shampoing, un après-shampoing, une huile, un lait, une pommade, une pâte, une mousse, une émulsion, un hydrogel, un gel douche, un masque, une laque, un spray, une cire, encore préférentiellement il s'agit d'une crème, d'un sérum ou d'une lotion.

De manière préférentielle, l'extrait est particulièrement adapté pour la formulation de composition dite neutre et douce pour le respect de la glande séborrhée, notamment de la peau incluant le cuir chevelu.

Comme précédemment mentionné, l'extrait *de Bixa orellana* selon la présente invention est préférentiellement utilisé sous forme de compositions cosmétiques, ou pharmaceutiques, préférentiellement dermatologiques.

Les compositions selon l'invention peuvent contenir tout solvant approprié et/ou tout véhicule approprié et/ou tout excipient approprié, éventuellement en combinaison avec d'autres composés d'intérêts.

De ce fait, pour ces compositions, l'excipient contient par exemple au moins un composé choisi parmi le groupe consistant en les conservateurs, les émollients, les émulsifiants, les tensioactifs, les hydratants, les épaississants, les conditionneurs, les agents matifiants, les stabilisants, les antioxydants, les agents de texture, les agents de brillance, les agents filmogènes, les solubilisants, les pigments, les colorants, les parfums et les filtres solaires. Ces excipients sont de préférence choisis parmi le groupe consistant en les acides aminés et leurs dérivés, les polyglycérols, les esters, les polymères et dérivés de cellulose, les dérivés de Lanoline, les phospholipides, les lactoferrines, les lactoperoxidases, les stabilisants à base de sucrose, les vitamines E et ses dérivés, les cires naturelles et synthétiques, les huiles végétales, les triglycérides, les insaponifiables, les phytosterols, les esters végétaux, les silicones et ses dérivés, les hydrolysats de protéines, l'huile de Jojoba et ses dérivés, les esters lipo/hydrosolubles, les betaines, les aminoxides, les extraits de plantes les esters de Saccharose, les dioxydes de Titane, les glycines, et les parabens, et encore de préférence parmi le groupe consistant en le butylène glycol, le stéareth-2, le stéareth-21, le glycol-15 stéaryl éther, le cétéaryl alcool, le phénoxyéthanol, le méthylparaben, l'éthylparaben, le propylparaben, le butylparaben, le butylène glycol, les tocopherols naturels, la glycérine, le dihydroxycetyl sodium phosphate, l'isopropyl hydroxycétyl éther, le glycol stéarate, le triisononanoin, l'octyl cocoate, le polyacrylamide, l'isoparaffine, le laureth-7, un carbomer, le propylène glycol, le glycérol, le bisabolol, une diméthicone, l'hydroxyde de sodium, le PEG 30-dipolyhydroxystérate, les caprique/caprylique triglycérides, le cétéaryl octanoate, le dibutyl adipate, l'huile de pépin de raisin, l'huile de jojoba, le sulfate de magnésium, l'EDTA, une cyclométhicone, la gomme de xanthane, l'acide citrique, le lauryl sulfate de sodium, les cires et les huiles minérales, l'isostéaryl isostéarate, le dipélargonate de propylène glycol, l'isostéarate de propylène glycol, le PEG 8, Beeswax, les glycérides d'huile de coeur de palme hydrogénée, les glycérides d'huile de palme hydrogénée, l'huile de lanoline, l'huile de sésame, le cétyl lactate, le lanoline alcool, l'huile de ricin, le dioxyde de titane, le lactose, le saccharose, le polyéthylène basse densité, une solution isotonique salée.

De nombreux ingrédients cosmétiquement actifs sont connus par l'homme du métier pour améliorer la santé et/ou l'apparence physique de la peau. L'homme du métier sait formuler les compositions cosmétiques ou dermatologiques pour obtenir les meilleurs effets. D'autre part les composés décrits dans la présente invention peuvent avoir un effet de synergie lorsqu'ils sont combinés les uns aux autres. Ces combinaisons sont également couvertes par la présente invention. Le CTFA Cosmetic Ingrédient Handbook, Second Edition (1992) décrit différents ingrédients cosmétiques et pharmaceutiques utilisés couramment dans l'industrie cosmétique et pharmaceutique, qui sont en particulier adaptés à une utilisation topique. Des exemples de ces classes d'ingrédients comprennent, sans en être limité les composés suivants: abrasif, absorbants, composé à but esthétique tel que les parfums, les pigments, les colorants, les huiles essentielles, les astringents, (par exemple: l'huile de clou de girofle, menthol, camphre, l'huile d'eucalyptus, eugénol, menthyl lactate, distillat d'hamélis), les agents anti-acné, les agents anti-floculants, les agents antimousse, les agents antimicrobiens (par exemple: iodopropyl butylcarbamate), les antioxydants, les liants, les additives biologiques, les agents tampon, les agents gonflants, les agents chélatants, les additifs, les agents biocides, les dénaturants, les épaississants, et les vitamines, et les dérivés ou équivalents de ceux-ci, les matériaux formant des films, les polymères, les agents opacifiants, les ajusteurs de pH, les agents réducteurs, les agents dépigmentants ou éclaircissants (par exemple : hydroquinone, acide kojique, acide ascorbique, magnésium ascorbyl phosphate, ascorbyl glucosamine), les agents de conditionnement (par exemple : les humectants).

De manière particulièrement avantageuse, l'extrait *de Bixa orellana* selon l'invention peut être utilisé, éventuellement dans une composition cosmétique ou pharmaceutique, préférentiellement dermatologique, préférentiellement celles précédemment décrites, comme seul agent actif, notamment comme seul agent actif séborégulateur ou en combinaison avec un ou plusieurs autres agents actifs choisis parmi :
1) un autre agent séborégulateur cosmétique et/ou dermatologique, préférentiellement, la sarcosine tel que commercialisé par la Demanderesse sous le nom MAT-XS ^{™} Clinical, un extrait d'*Orthosiphon stamineus* tel que commercialisé par la Demanderesse sous le nom MAT-XS ^{™} Bright, le gluconate de zinc, le salicylate de zinc, l'acide azélaique et/ou leurs dérivés, et/ou leurs mélanges, avantageusement en combinaison avec le gluconate de zinc.
   et/ou
2) un agent à propriétés complémentaires choisis parmi les agents exfoliants et/ou kératolytiques, les agents absorbeurs de sébum, les agents ayant une action sur le microbiote, les agents comédolytiques et/ou les agents hydratants.

En particulier, on peut citer comme exemple préférentiel :
- d'agent exfoliant et/ou kératolytique : les alpha-hydroxy-acides (AHA) notamment l'acide salicylique, éventuellement en combinaison des protéines d'acacia, l'acide malique, éventuellement en association avec des protéines d'amande, l'acide glycolique ; l'acide lactique et/ou leurs dérivés; et/ou leurs mélanges
- d'agent absorbeur de sébum: un talc et/ou un polymère absorbant
- d'agent ayant une action sur le microbiote notamment cutané:
- les agents antibactériens décrits dans la demande de brevet FR2863893, et en particulier un extrait de Peumus boldus, un tel extrait étant notamment commercialisé par la Demanderesse sous le nom Betapur ^{™} et/ou un agent antibiotique local, en particulier l'érythromycine et/ou le phosphate de clindamycine.
- les agents équilibrants la flore microbienne tels qu'un mélange de pullulan, alginate de sodium et hyaluronate de sodium commercialisé par la Demanderesse sous le nom PatcH20 ^{™} notamment associé à la sérine, tréhalose et urée et décrit dans la demande de brevet WO2014027163A2, et/ou un extrait de Saccharomyces cerevisiae pour augmenter la flore microbienne commensale cutanée et/ou mucosale commercialisé par la Demanderesse sous le nom Relipidium^{™}.
- d'agent comédolytique : l'acide rétinoique et un de ses dérivés tels que isotrétinoine, adapalène et/ou acide 13cis retinoique et le péroxyde de benzoyle;
- d'agent hydratants : un ou plusieurs agents favorisant l'hydratation tels qu'un polysaccharide extrait de graines de *Cassia angustifolia* commercialisée sous le nom de Hyalurosmooth^{™} par la demanderesse, ou un agent choisi parmi une des combinaisons contenant du pullulan, du hyaluronate de sodium et de l'alginate de sodium commercialisé sous le nom de PatcH2O^{™} par la demanderesse ou encore un ou plusieurs des composés du facteur natural d'hydratation (Natural Moisturizing Factor) ou un extrait naturel de miel commercialisé par la demanderesse sous le nom de Melhydran^{™} et/ou un composé de la famille des glucosyl glycérides, en particulier l'hexosyl glycéride, un extrait de péricarpe de *Litchi chinensis* sous le nom Litchiderm^{™} par la déposante ;

De manière préférentielle la composition cosmétique contenant l'extrait selon l'invention contiendra un autre agent séborégulateur choisi parmi la sarcosine et un extrait *d'Orthosiphon stamineus*, le gluconate de zinc et leurs mélanges, un agent hydratant choisi parmi extrait de graines de *Cassia angustifolia*, l'hexosyl glycéride, un mélange contenant du pullulan, du hyaluronate de sodium et de l'alginate de sodium et leurs mélanges. De manière avantageuse, la composition contenant l'extrait selon l'invention contiendra en outre un agent agissant sur la flore bactérienne choisi parmi un extrait de *Peumus boldus*, un mélange de pullulan, alginate de sodium et hyaluronate de sodium, un extrait de Saccharomyces cerevisiae et leurs mélanges.

Les compositions cosmétiques contenant l'extrait selon l'invention pourront contenir les actifs classiques des compositions cosmétiques notamment choisis parmi :
- un agent stimulant la synthèse de fibronectine, en particulier un extrait de mais, un tel extrait étant notamment commercialisé par la Demanderesse sous le nom Deliner^{™}
- un agent de protection du facteur de croissance des fibroblastes (FGF2) de la matrice extracellulaire contre sa dégradation et/ou sa dénaturation, notamment un extrait d'*Hibiscus Abelmoscus* tel que décrit dans la demande de brevet au nom de la Demanderesse déposée sous le numéro FR0654316 et/ou un agent de stimulation de croissance des fibroblastes par exemple un extrait de soja fermenté contenant des peptides, connu sous le nom de Phytokine^{™} commercialisé par la Demanderesse et également décrit dans la demande de brevet EP1119344 B1 (Laboratoires Expanscience), et préférentiellement une combinaison de ces deux extraits

- un agent stimulant la synthèse de laminine, en particulier un extrait de malt modifié par biotechnologie, un tel extrait étant notamment commercialisé par la Demanderesse sous le nom Basaline ^{™} ;
- un agent stimulant l'expression et/ou l'activité de la Hyaluronane synthase 2 (HAS2) tels que les extraits végétaux décrits dans la demande de brevet FR2 893 252 A1 et en particulier un extrait aqueux de Galanga (*Alpinia galanga) ;*
- un agent stimulant la synthèse de lysyl oxydase like (LOXL) tel que ceux décrits dans la demande de brevet FR2855968, et en particulier un extrait d'aneth ;
- un ou plusieurs agents anti-pollution tels qu'un extrait de feuilles d'*Argania spinosa* commercialisé sous le nom d'Arganyl^{™} ou un extrait de graines de *Moringa oleifera* commercialisé sous le nom de Purisoft^{™} par la demanderesse ou encore un extrait de racine d'*Eperua falcata* commercialisé sous le nom de Eperuline^{™}
- un agent stimulant la synthèse d'ATP intracellulaire, notamment un extrait d'algue *Laminaria digitata ;*
- un agent à action globale anti-age, notamment anti-taches pigmentaires en particulier la niacinamide ou vitamine B3;
et l'un quelconque de leurs mélanges.

Selon la présente invention, l'utilisation de l'extrait *de Bixa orellana* selon l'invention est particulièrement avantageuse en ce qu'elle permet une action séborégulatrice complète, efficace et durable sur tout type de peaux et/ou cheveux, en particulier les peaux caucasiennes ou africaine ou asiatiques et/ou les cheveux caucasiens, africains ou asiatiques, en particulier les peaux caucasiennes ou asiatiques et/ou les cheveux caucasiens ou asiatiques, plus particulièrement les peaux asiatiques et/ou les cheveux asiatiques.

De manière préférentielle, l'extrait de *Bixa orellana* selon l'invention, préférentiellement sous la forme d'une composition cosmétique selon l'invention est appliqué sur au moins une zone du corps où la sécrétion de sébum est inconfortable et/ou inesthétique et/ou désagréable, cette ou ces zones étant préférentiellement une surface du corps choisie parmi la peau du visage, incluant le front, les joues, le nez, les tempes, la zone T (front, nez et menton) le conduit auditif externe et /ou le menton, le cuir chevelu, du cou, le dos, les épaules, les avant-bras, du thorax, les mains, et/ou le buste.

La présente description décrit également un procédé de soin cosmétique (qui ne fait pas partie de l'invention) dans lequel, l'extrait de *Bixa orellana* selon l'invention est appliqué sur au moins une partie du corps, préférentiellement une surface choisie parmi la peau du visage, incluant le front, les joues, le nez, les tempes, la zone T (front, nez et menton), le conduit auditif externe et /ou le menton, le cuir chevelu, du cou, du dos, des épaules, des avant-bras, du thorax, des mains, et/ou du buste, préférentiellement pour prévenir et/ou réduire la sécrétion de sébum et/ou pour prévenir et/ou réduire les effets inesthétiques et/ou inconfortables et/ou désagréables de la sécrétion de sébum.

De manière avantageuse, l'extrait *de Bixa orellana* selon l'invention, préférentiellement sous la forme d'une composition cosmétique selon l'invention est utilisé en application topique régulière et préférentiellement au moins une fois par jour, avantageusement deux fois par jour, pendant au moins 10 jours, préférentiellement pendant 20 jours, et encore préférentiellement pendant au moins 40 jours. De manière préférentielle, la composition cosmétique est appliquée sur la peau sans rinçage en massant.

De manière avantageuse, il est également décrit une méthode de traitement cosmétique (qui ne fait pas partie de l'invention) pour prévenir et/ou réduire la sécrétion de sébum d'un individu qui en a besoin/qui le souhaite comprenant les étapes :
a) L'identification sur l'individu d'une zone de peau dont on souhaite diminuer la sécrétion de sébum et/ou les manifestations inesthétiques et/ou désagréables et/ou inconfortables liées à la sécrétion de sébum, et
b) L'application topique sur cette zone de peau d'une composition cosmétique contenant l'extrait de *Bixa orellana* selon l'invention en une quantité efficace pour prévenir et/ou réduire la sécrétion de sébum sur cette zone de peau, à savoir en une teneur d'extrait comprise entre 1×10⁻⁴% à 10% en poids, préférentiellement de 1×10⁻⁴% à 5% en poids, encore avantageusement de 1×10⁻³% à 3% en poids, encore préférentiellement de 0,001% et 0,1 % en poids, par rapport au poids total de la composition.

L'extrait de *Bixa orellana* selon l'invention peut être utilisé pour le traitement et/ou la prévention d'une pathologie associée à une hyperproduction de sébum, en particulier les hyperséborrhées pathologiques, l'eczéma séborrhéique et/ou l'hypersécrétion du nourrisson et/ou l'une quelconque de leurs combinaisons.

De manière préférentielle, l'extrait de *Bixa orellana* selon l'invention, préférentiellement sous la forme d'une composition pharmaceutique selon l'invention est appliquée sur au moins une zone du corps où la sécrétion de sébum est excessive, induit et/ou est associée à au moins une pathologie, en particulier sur au moins une zone du corps présentant une hyperséborrhée pathologique, cette ou ces zones étant préférentiellement une surface du corps choisie parmi la peau du visage, incluant le front, les joues, le nez, les tempes, la zone T (front, nez et menton), le conduit auditif externe et /ou le menton, le cuir chevelu, le cou, le dos, les épaules, les avant-bras, le thorax, les mains, et/ou le buste.

Dans un mode de réalisation préférentiel de l'invention, l'extrait selon l'invention est présent dans la composition pharmaceutique en une teneur comprise entre 1×10⁻⁴% à 10% en poids, préférentiellement de 1×10⁻⁴% à 5% en poids, encore avantageusement de 1×10⁻³% à 3% en poids, encore préférentiellement de 0,001% et 0,1 % en poids, par rapport au poids total de la composition.

D'autres buts, caractéristiques et avantages de l'invention apparaîtront clairement à l'homme de l'art suite à la lecture de la description explicative qui fait référence à des exemples qui sont donnés seulement à titre d'illustration et qui ne sauraient en aucune façon limiter la portée de l'invention.
Les exemples font partie intégrante de la présente invention et toute caractéristique apparaissant nouvelle par rapport à un état de la technique antérieure quelconque à partir de la description prise dans son ensemble, incluant les exemples, fait partie intégrante de l'invention dans sa fonction et dans sa généralité.
Ainsi, chaque exemple a une portée générale.

D'autre part, dans les exemples, tous les pourcentages sont donnés en poids, sauf indication contraire, la température est exprimée en degré Celsius sauf indication contraire, et la pression est la pression atmosphérique, sauf indication contraire.

### EXEMPLE

### EXEMPLE 1 : Différentes méthodes de préparation de l'extrait de Bixa orellana selon l'invention

***Exemple 1a) :*** Une quantité de 10% (p/p par rapport au mélange total eau et graines) de graines broyées de *Bixa orellana* a été mise à macérer dans l'eau comme solvant pendant une heure à une température de 80°C. L'extrait brut a été centrifugé, décanté puis filtré puis atomisé en présence de maltodextrine, en quantité finale de maltodextrine de 60% en poids par rapport au poids total de l'extrait final.

***Exemple 1b) :*** Une quantité de 10% en poids de graines broyées (par rapport au poids total eau et graines) a été mise à macérer dans l'eau pendant 2 heures à une température de 20°C. L'extrait brut a été centrifugé, décanté puis filtré.

***Exemple 1c)*** : Une quantité de 20% en poids de graines broyées (par rapport au poids total eau et graines) a été mise à macérer dans l'eau pendant de 2 heures à une température de 20°C. L'extrait a été ensuite centrifugé, décanté, filtré.

***Exemple 1d)*** : Une quantité de 10% en poids de graines broyées (par rapport au poids total des graines et du solvant) a été mise à macérer dans un solvant constitué d'un mélange éthanol : eau (80 :20 ; v/v), à une température de 80°C durant une période de 1 heure. L'extrait a été ensuite centrifugé, décanté, filtré puis atomisé en présence de maltodextrine, en quantité finale de maltodextrine de 80% en poids par rapport au poids total de l'extrait final.

***Exemple 1e) comparatif (qui ne fait pas partie de l'invention) :*** Une quantité de 10% en poids de feuilles broyées (par rapport au poids total eau et feuilles) a été mise à macérer dans de l'eau pendant une heure à une température de 80°C. L'extrait brut a été centrifugé, décanté puis filtré puis atomisé en présence de maltodextrine, en quantité finale de maltodextrine de 60% en poids par rapport au poids total de l'extrait final

### EXEMPLE 2 : Propriétés d'un extrait de Bixa orellana selon l'invention sur la sécrétion de sébum.

### Méthode

Des sébocytes humains ont été ensemencés à 2500 cellules/centimètre ² dans un milieu de culture complet (DMEM/HAM F12 avec du sérum foetal bovin (FCS) à 10 % (p/p dans le milieu)et incubés pendant 5 jours à 37°C, CO2 à 5 % d'humidité relative plus de 95 % . Alors le milieu de culture de croissance a été remplacé par un milieu de culture standard (DMEM) contenant du sérum humain (HS) à 1 % ou IGF1 à 100 ng/ml. L'extrait de *Bixa orellana* obtenu à l'exemple 1a) tel qu'obtenu avant l'étape de mélange avec la maltodextrine a été incubé pendant 5 jours à 37°C à 0,02% ou 0,05% en poids/poids par rapport au mélange milieu et l'extrait. Les cellules sont rincées en tampon PBS (phosphate buffer saline) et fixées par une solution de formaldéhyde. Le nombre de sébocytes est déterminé par le maculage d'ADN avec le réactif de Hoechst et la fluorescence est enregistrée à 465 nm (l'excitation à 356 nm) alors que la quantité de lipides cellulaire est déterminée avec une coloration au Nile Red (11). La fluorescence des lipides totaux est mesurée à une longueur d'onde de 625 nm (l'excitation à 520 nm) alors que la fluorescence des lipides neutres est mesurée à une longueur d'onde de 530 nm (l'excitation à 475 nm). Les résultats sont exprimés en pourcentage par rapport au contrôle sans l'extrait de *Bixa orellana* (le moyen d'expression basal avec HS à 1 % ou IGF1 à 100ng/mL) et présentés comme une moyenne +/-l'écart-type (SD). Les statistiques ont été évalués avec le logiciel SigmaPlot^{™}.

### Résultats

### 1. Activation avec HS 1%

### Sébocytes asiatiques, 25 ans.

### N=3

| | sans HS | **HS à 1%** | |
|---|---|---|---|
| | | Contrôle | Extrait à 0,02% |
| Nombre de cellules (ADN) | 61+/-27 | **100+/-6** | 83+/-4 |
| Lipides totaux | 50+/-14 | **100+/-4** | 84+/-7 p<0.001 |
| Lipides neutres | 74+/-24 | **100+/-5** | 76+/-24 p<0.001 |

L'extrait de *Bixa orellana* selon l'invention a diminué de façon significative la prolifération des sébocytes et la synthèse des lipides totaux dans des sébocytes traités avec HS 1%.

### 2. Activation avec IGF-1 100 ng/mL.

### 2.1. Sébocytes Caucasien, 25 ans.

| | sans IGF | **IGF à100 ng/mL** | |
|---|---|---|---|
| | | Contrôle | Extrait 0,05% |
| Nombre de cellules (ADN) | 81+/-6 | **100+/-8** | 48+/-3 p< 0,001 |
| Lipides totaux | 77+/-11 | **100+/-7** | 33+/-3 p< 0,001 |
| Lipides neutres | 94+/-1 | **100+/-3** | 86+/-2 p< 0,01 |

L'extrait de *Bixa orellana* selon l'invention à une concentration de 0,05% a diminué de façon significative la prolifération des sébocytes et la synthèse des lipides totaux et neutres dans des sébocytes traités avec IGF-1. Des résultats similaires ont été obtenus à 0,02% d'extrait selon l'invention.

### 2.2. Sébocytes asiatiques, 25 ans.

| | sans IGF | **IGF à 100 ng/mL** | |
|---|---|---|---|
| | | Contrôle | Extrait 0,05% |
| Nombre de cellules (ADN) | 89+/-5 | **100+/-8** | 69+/-9 p< 0,05 |
| Lipides totaux | 88+/-10 | **100+/-11** | 53+/-11 p<0,01 |

L'extrait de *Bixa orellana* selon l'invention à une concentration de 0,05% diminue de façon significative la prolifération des sébocytes et la synthèse des lipides totaux dans des sébocytes traités avec IGF-1.

### EXEMPLE 3 : Induction de la protéine IGF1- Binding protéine : IGFBP3.

La protéine BP3 est dédiée à bloquer la liaison d'IGF-1 sur son récepteur respectif IGF1R, elle va donc bloquer la voie IGF1- IGF1R, donc enrayer l'hyperprolifération et différentiation des kératinocytes. En activant cette protéine, l'extrait de *Bixa orellana* freine l'hyperkératinisation observée dans les peaux grasses. L'extrait de *Bixa orellana* selon l'invention augmente la synthèse de la protéine IGFBP3.

### Matériel et Méthode

Des kératinocytes humains normaux sont cultivés jusqu'à 70% de confluence, puis traités pendant 24H avec IGF-1 100 ng/ml ou l'extrait de *Bixa orellana* obtenu selon l'exemple 1a) avant mélange avec la maltodextrine et atomisation à 0.1% ou 0.05% (p/p dans le milieu total) pendant 24H. Les cellules sont lysées pour le dosage de l'ADN, et le surnageant est utilisé pour la quantification de la protéine IGFBP3 en ELISA selon le protocole du fournisseur du Kit ELISA (LSBio Human IGFBP3 ELISA Kit ref LS-F24538 lot 103180).

### Résultats

Les résultats sont rapportés aux cellules non traitées. Un test statistique t-test est réalisé, les moyennes sont comparées.

| | Moyenne | écart | Statistique |
|---|---|---|---|
| Non traité | 100 | 19,3937 | |
| IGF-1 | 128,195 | 25,6252 | NS |
| extrait 0,1% | 147,769 | 25,3724 | P<0,01 |
| extrait 0,05% | 174,824 | 24,9337 | P <0,001 |

L'extrait de *Bixa orellana* selon l'invention a augmenté la quantité de protéine IGFBP3

### EXEMPLE 4 : Augmentation de la synthèse de collagène IV par l'extrait selon l'invention.

### Méthode :

Des kératinocytes humains dits normaux issus du sein d'un donneur sain de 24 ans ont été cultivés dans un milieu défini (KSFM) durant une période de 48 heures jusqu'à confluence (100%) en présence de 3 concentrations finales différentes de l'extrait de *B.orellana* préparé selon l'exemple 1a) avant le mélange et l'atomisation puis le milieu cellulaire est éliminé. Le tapis cellulaire obtenu est ensuite lysé avec une solution d'hydroxyde d'ammonium. Une partie du lysat a été utilisée pour le dosage de l'ADN. Le dosage du collagène IV a été effectué avec un anticorps anti-collagène IV (Acris, R1041) dilué au 1/2500 dans une solution de tampon PBS contenant de la Sérum Albumine Bovine (SAB). Après une période de 60 minutes, l'anticorps secondaire (PerkinElmer, AD0105) dilué au 1/25000 est appliqué durant une période de 1 heures à l'obscurité. La fluorescence a été mesurée (ENVision, PerkinElmer).

### Résultats

Les résultats de fluorescence ont été normalisés par rapport à la fluorescence obtenue avec le même milieu cellulaire en l'absence de l'extrait de *B. orellana* (Contrôle) et ont été rapportés au pourcentage d'ADN obtenu dans chaque condition. Les résultats présentés correspondent à la moyenne des essais. (ET : Ecart-type).

| | Moyenne | Ecart Type | statistique |
|---|---|---|---|
| Non traité | 100,00 | 7,53 | |
| extrait 0,025 % | 144,08 | 8,67 | p<0,001 |
| Extrait 0,05% | 157,83 | 7,77 | p<0,001 |
| Extrait 0,1% | 159,83 | 14,64 | p<0,001 |

L'extrait de *Bixa orellana* selon l'invention augmente la synthèse de Collagène IV dans les kératinocytes et permet donc de resserrer les pores de la peau.

### EXEMPLE 5 : Etude clinique d'un extrait de Bixa orellana selon l'invention sur la sécrétion de sébum.

### Méthode :

Une étude clinique a été réalisée en double aveugle, en hémi- visage, une partie avec le placébo et une partie avec l'extrait de *Bixa orellana* selon l'invention à 0.25% tel qu'obtenu dans l'exemple 1a) et formulé dans la composition décrite ci-dessous (Formule selon l'invention).

L'étude a été réalisée sur 35 femmes asiatiques agées de 20 à 45 ans, avec un indice lipidique mesuré par un sébumètre (Sebumeter^{™} SM 815) ≥ 150 microgrammes de sébum par cm² (peau très grasse) et incluant un maximum de 10 volontaires avec un indice lipidique mesuré par un sébumètre (Sebumeter^{™} SM 815) allant de 120 à 149 microgrammes de sébum par cm² (peau grasse). Le traitement a été appliqué 2 fois par jour pendant 56 jours.

| Marque | INCI | Formule Placebo (% p/p) | Formule selon l'invention (%p/p) |
|---|---|---|---|
| Eumulgin^{®} SG | Sodium stearoyl glutamate | 0,5 | 0,5 |
| Solution acide citrique 10% | Citric acid , aqua | 0,7 | 0,65 |
| Cosmedia ^{®} SP | Sodium polyacrylate | 0,7 | 0,7 |
| Cutina^{®} PES | Pentaerythrityl distearate | 1 | 1 |
| Glycérine 99-5 | Glycerin | 2 | 2 |
| Elestab^{™} 388 | Propylène glycol, phenoxyethanol, chlorphenesin, methylparaben | 2,5 | 2,5 |
| Emulgade^{™} Sucro Plus | Sucrose polystearate, cetyl palmitate | 3 | 3 |
| Miritol^{™} 318 | Caprylic /capric triglyceride | 3 | 3 |
| Cetiol ^{®} C-5-C | Coco-caprilate/caprate | 3 | 3 |
| Cetiol ^{®} CC | Caprylyl carbonate | 3 | 3 |
| Extrait selon l'invention selon ex 1a) | *Bixa orellana* | 0 | 0,25 |
| Eau | Aqua | Qsp | qsp |

Les compositions ont été réalisées par mélange selon les méthodes connues par l'Homme du métier.

Des prélèvements à l'aide de patch Sébutape^{®} ont été effectués et ont permis de mesurer l'activité des glandes sébacées en mesurant la quantité de sébum avant (D0) et après traitement à 28 et 56 jours. La taille des pores sur les joues, évaluée au départ entre 2 et 4, a été évaluée par un calcul clinique réalisé par une personne référente formée. L'hydratation de la peau a été mesurée par la méthode de Cornéométrie. Un questionnaire a été rempli par les volontaires à 28 et 56 jours.

### Résultats :

A 28 jours de traitement avec l'extrait de *Bixa orellana* selon l'invention à 0,25%, le nombre de spots (sébum sécrété) diminue significativement de 22% versus D0, et significativement de 14% versus placebo.

A 56 jours de traitement avec l'extrait de *Bixa orellana* selon l'invention à 0,25%, le nombre de spots (sébum sécrété) diminue significativement de 37% versus D0, et significativement de 25% versus placebo.

A 28 jours avec l'extrait de *Bixa orellana* selon l'invention à 0.25%, la surface des spots diminue significativement de 46% versus D0, et significativement de 31% versus placebo.

A 56 jours de traitement avec l'extrait de *Bixa orellana* selon l'invention à 0,25%, le nombre de spots (sébum sécrété) diminue significativement de 62% versus D0, et significativement de 36% versus placebo.

A 28 jours de traitement avec l'extrait de *Bixa orellana* selon l'invention à 0,25%, la taille des pores diminue visuellement et significativement de 3% versus D0. A 56 jours de traitement avec l'extrait de *Bixa orellana* selon l'invention à 0,25%, la taille des pores diminue significativement de 9% versus D0 et de 3% versus placebo.

Après 28 et 56 jours de traitement, l'extrait de *Bixa orellana* selon l'invention à 0,25% augmente de manière significative la valeur du cornéométre de 3% versus D0.

### EXEMPLE 6 : composition cosmétique selon l'invention

L'extrait utilisé est celui obtenu dans l'exemple 1a) (après mélange avec maltodextrine et atomisation) sous forme de poudre.

### Exemple 6a) : Soin tonique pour affiner le grain de peau

| Phase | Dénomination | Quantité (% en poids total) |
|---|---|---|
| A | Eau | 87,65 |
| A | Glycérine | 4,00 |
| A | EDTA disodium | 0,05 |
| A | Conservateur | q.s. |
| A | Polysorbate 20 | 1,00 |
| A | Poloxamer 184 | 2,00 |
| A | Gluconate de zinc | 0,05 |
| B | eau | 3 |
| B | Extrait de *Bixa orellana* selon l'exemple1a) | 0,25, |
| B | Ajusteur de pH (acide citrique) | q.s. |
| C | Cocoate de glycéryle PEG-7 | 0,5 |
| C | Parfum | 0,1 |

Le soin tonique est préparé par les méthodes usuelles dans le domaine bien connu de l'homme du métier, en mélangeant les 3 phases et en ajustant la composition à un pH de 5,1.

### Exemple 6b) : Crème matifiante

| Phase | Dénomination | Quantité (% en poids total) |
|---|---|---|
| A | Polystearate sucrose, palmitate cetyl | 3,00 |
| A | Distearate pentaerythrityl | 1,00 |
| A | Triglyceryle Caprylic/capric | 3,00 |
| A | Coco-caprylate/caprate | 3,00 |
| A | Carbonate de Dicaprylyl | 3,00 |
| A | Polyacrylate de sodium | 0,70 |
| B | eau | 80,90 |
| B | glycérine | 2,00 |
| B | Glutamate stearoyl sodium | 0,50 |
| B | conservateur | q.s. |
| C | Extrait de *Bixa orellana* selon l'exemple 1a) | 0,25 |
| C | Eau | 2,00 |
| D | Parfum | q.s. |
| E | Ajusteur de pH (acide citrique) | q.s. |

La crème est préparée par les méthodes usuelles dans le domaine bien connu de l'homme du métier, en mélangeant les phases A et B préalablement chauffées à 75°C, puis en ajoutant les phases C et D en mélangeant et en ajustant la composition avec la phase E à un pH de 6,2 et à la viscosité de 15 000 mPas (mesurée avec un appareil Brookfield (RVT ; 23°C, spindle TC ; 20tours par min).

### Exemple 6c) : shampoing

| Phase | Dénomination | Quantité (% en poids total) |
|---|---|---|
| A | Eau | 60,3 |
| A | Gomme xanthane | 1,2 |
| B | Décyl glucoside | 14 |
| B | Dicaprylyl éther, décyl glucoside, oléate de glycéryle | 5 |
| B | Sodium cocoyle glutamate | 12 |
| B | Coco-glucoside, oléate de glycéryle | 2 |
| B | Glycérine | 3 |
| B | Conservateur | q.s. |
| C | Ajusteur de pH (acide citrique) | q.s. |
| D | Parfum | 0,5 |
| D | Extrait de *Bixa orellana* selon l'exemple 1a) | 0,01-10 |

Le shampoing est préparé par les méthodes usuelles dans le domaine bien connu de l'homme du métier, en mélangeant les 4 phases et en ajustant la composition à un pH de 5,2 et à la viscosité de 2200 mPas (mesurée avec un appareil Brookfield (RVT ; 23°C, spindle 5 ; 50tours par min).

## Revendications

1. Utilisation cosmétique non thérapeutique d'un extrait hydrosoluble de *Bixa orellana* pour prévenir et/ou réduire la sécrétion de sébum et/ou pour prévenir et/ou réduire les manifestations inesthétiques et/ou désagréables et/ou inconfortables liées à la sécrétion de sébum **caractérisée en ce que** l'extrait de *Bixa orellana* est un extrait de graine obtenu par extraction aqueuse.

2. Utilisation selon la revendication précédente, **caractérisée en ce que** les manifestations inesthétiques et/ou désagréables et/ou inconfortables de la sécrétion de sébum sont la visibilité des pores de la peau et/ou la formation de points noirs et/ou l'aspect luisant et/ou gras de la peau et/ou des cheveux et/ou l'aspect épais de la peau.

3. Utilisation selon la revendication précédente, **caractérisée en ce que** l'extrait de *Bixa orellana* est atomisé en présence d'une concentration en poids de maltodextrine comprise entre 20% et 90%, préférentiellement entre 40 et 80%, encore préférentiellement 60% par rapport au poids total de la poudre obtenue.

4. Utilisation selon l'une des revendications précédentes, **caractérisée en ce que** l'extrait de *Bixa orellana* se trouve sous la forme d'une composition cosmétique comprenant en outre un excipient cosmétiquement acceptable.

5. Utilisation selon la revendication 4, **caractérisée en ce que** la composition cosmétique est destinée à une application par voie topique sur la peau notamment le cuir chevelu, et/ou les cheveux.

6. Utilisation selon la revendication précédente, **caractérisé en ce que** l'extrait de *Bixa orellana* est présent dans la composition cosmétique en une teneur comprise entre 1×10⁻⁴% à 10% en poids, préférentiellement de 1×10⁻⁴% à 5% en poids, encore avantageusement de 1×10⁻³% à 3 % en poids, encore préférentiellement de 0,001% et 0,1 % en poids, par rapport au poids total de la composition.

7. Utilisation selon l'une des revendications 5 ou 6 dans laquelle la peau est normale, grasse, très grasse et/ou mixte et/ou les cheveux sont normaux et/ou gras.

8. Utilisation selon l'une des revendications 5 à 7 dans laquelle la peau est caucasienne ou africaine ou asiatique et/ou les cheveux sont caucasiens, africains ou asiatiques.

9. Utilisation selon l'une quelconque des revendications 4 à 8, **caractérisé en ce que** la composition cosmétique est choisi dans le groupe constitué par un sérum, une lotion, une crème, un shampoing, un après-shampoing, une huile, un lait, une pommade, une pâte, une mousse, une émulsion, un hydrogel, un gel douche, un masque, une laque, un spray, une cire, avantageusement il s'agit d'un crème, d'un sérum ou d'une lotion.

10. Utilisation selon l'une quelconque des revendications 1 à 9, **caractérisé en ce que** l'extrait de *Bixa orellana* est appliqué sur au moins une zone du corps où la sécrétion de sébum est inconfortable et/ou inesthétique et/ou désagréable, cette ou ces zones étant préférentiellement une surface du corps choisie parmi la peau du visage, incluant le front, les joues, le nez, les tempes, la zone T (front, nez et menton), le conduit auditif externe et/ou le menton, le cuir chevelu, le cou, le dos, les épaules, les avant-bras, le thorax, les mains, et/ou le buste.

11. Extrait hydrosoluble de *Bixa orellana* pour son utilisation dans le traitement et/ou la prévention d'une pathologie associée à une hyperproduction de sébum choisie parmi les hyperséborrhées pathologiques et l'eczéma séborrhéique et/ou l'une quelconque de leurs combinaisons, **caractérisé en ce que** l'extrait de *Bixa orellana* est un extrait de graine obtenu par extraction aqueuse.

12. Extrait de *Bixa orellana* pour son utilisation selon la revendication précédente **caractérisé en ce que** l'extrait est tel que défini selon la revendication 3.

13. Extrait de *Bixa orellana* pour son utilisation selon l'une quelconque des revendications 11 ou 12, **caractérisé en ce que** l'extrait est présent dans la composition pharmaceutique en une teneur comprise entre 1×10⁻⁴% à 10% en poids, préférentiellement de 1×10⁻⁴% à 5% en poids, encore avantageusement de 1×10⁻³% à 3% en poids, encore préférentiellement de 0,001% et 0,1 % en poids, par rapport au poids total de la composition.

## Patentansprüche

1. Nichttherapeutische kosmetische Verwendung eines wasserlöslichen Extrakts von *Bixa orellana* zum Verhindern und/oder Verringern von Talgsekretion und/oder zum Verhindern und/oder Verringern der mit der Talgsekretion einhergehenden unästhetischen und/oder unangenehmen und/oder unkomfortablen Manifestationen, **dadurch gekennzeichnet, dass** es sich bei dem *Bixa* orellana-Extrakt um einen durch wässrige Extraktion erhaltenen Samenextrakt handelt.

2. Verwendung nach dem vorhergehenden Anspruch, **dadurch gekennzeichnet, dass** es sich bei den unästhetischen und/oder unangenehmen und/oder unkomfortablen Manifestationen von Talgsekretion um die Sichtbarkeit der Hautporen und/oder die Bildung von Mitessern und/oder das glänzende und/oder fettige Aussehen der Haut und/oder der Haare und/oder ein dickes Aussehen der Haut handelt.

3. Verwendung nach dem vorhergehenden Anspruch, **dadurch gekennzeichnet, dass** der *Bixa* orellana-Extrakt in Gegenwart einer Maltodextrin-Konzentration zwischen 20 Gew.-% und 90 Gew.-%, vorzugsweise zwischen 40 und 80 Gew.-%, ebenfalls bevorzugt 60 Gew.-%, bezogen auf das Gesamtgewicht des erhaltenen Pulvers, zerstäubt wird.

4. Verwendung nach einem der vorhergehenden Ansprüche, **dadurch gekennzeichnet, dass** der *Bixa* orellana-Extrakt in Form einer kosmetischen Zusammensetzung vorliegt, die außerdem einen kosmetisch akzeptablen Exzipienten umfasst.

5. Verwendung nach Anspruch 4, **dadurch gekennzeichnet, dass** die kosmetische Zusammensetzung für die topische Anwendung auf die Haut, insbesondere die Kopfhaut, und/oder die Haare bestimmt ist.

6. Verwendung nach dem vorhergehenden Anspruch, **dadurch gekennzeichnet, dass** der *Bixa* orellana-Extrakt in der kosmetischen Zusammensetzung in einem Gehalt zwischen 1×10⁻⁴ Gew.-% bis 10 Gew.-%, vorzugsweise von 1×10⁻⁴ Gew.-% bis 5 Gew.-%, ebenfalls vorteilhaft von 1×10⁻³ Gew.-% bis 3 Gew.-%, ebenfalls bevorzugt 0,001 Gew.-% und 0,1 Gew.-%, bezogen auf das Gesamtgewicht der Zusammensetzung, vorliegt.

7. Verwendung nach einem der Ansprüche 5 oder 6, wobei die Haut normal, fettig, sehr fettig und/oder gemischt ist und/oder die Haare normal und/oder fettig sind.

8. Verwendung nach einem der Ansprüche 5 bis 7, wobei die Haut kaukasisch, afrikanisch oder asiatisch ist und/oder die Haare kaukasisch, afrikanisch oder asiatisch sind.

9. Verwendung nach einem der Ansprüche 4 bis 8, **dadurch gekennzeichnet, dass** die kosmetische Zusammensetzung aus der Gruppe ausgewählt ist, die aus einem Serum, einer Lotion, einer Creme, einem Shampoo, einer Spülung, einem Öl, einer Milch, einer Salbe, einer Paste, einem Schaum, einer Emulsion, einem Hydrogel, einem Duschgel, einer Maske, einem Lack, einem Spray, einem Wachs besteht, es sich vorteilhafterweise um eine Creme, ein Serum oder eine Lotion handelt.

10. Verwendung nach einem der Ansprüche 1 bis 9, **dadurch gekennzeichnet, dass** der *Bixa* orellana-Extrakt auf mindestens einen Bereich des Körpers aufgetragen wird, in dem die Talgsekretion unkomfortabel und/oder unästhetisch und/oder unangenehm ist, wobei dieser Bereich bzw. diese Bereiche vorzugsweise eine aus der Haut des Gesichts, einschließlich der Stirn, der Wangen, der Nase, der Schläfen, der T-Zone (Stirn, Nase und Kinn), des äußeren Gehörgangs und/oder des Kinns, der Kopfhaut, dem Hals, dem Rücken, den Schultern, den Unterarmen, dem Brustkorb, den Händen und/oder dem Oberkörper ausgewählte Körperoberfläche ist bzw. sind.

11. Wasserlöslicher *Bixa* orellana-Extrakt zur Verwendung bei der Behandlung und/oder Vorbeugung einer mit einer Überproduktion von Talg einhergehenden Pathologie, die aus pathologischen Hyperseborrhoen und seborrhoischem Ekzem und/oder einer beliebigen ihrer Kombinationen ausgewählt ist, **dadurch gekennzeichnet, dass** es sich bei dem *Bixa* orellana-Extrakt um einen durch wässrige Extraktion erhaltenen Samenextrakt handelt.

12. *Bixa* orellana-Extrakt zur Verwendung nach dem vorhergehenden Anspruch, **dadurch gekennzeichnet, dass** es sich um einen Extrakt nach Anspruch 3 handelt.

13. *Bixa* orellana-Extrakt zur Verwendung nach einem der Ansprüche 11 oder 12, **dadurch gekennzeichnet, dass** der Extrakt in der pharmazeutischen Zusammensetzung in einem Gehalt zwischen 1×10⁻⁴ Gew.-% bis 10 Gew.-%, vorzugsweise von 1×10⁻⁴ Gew.-% bis 5 Gew.-%, ebenfalls vorteilhaft von 1×10⁻³ Gew.-% bis 3 Gew.-%, ebenfalls bevorzugt 0,001 Gew.-% und 0,1 Gew.-%, bezogen auf das Gesamtgewicht der Zusammensetzung, vorliegt.

## Claims

1. The non-therapeutic cosmetic use of a water-soluble extract of *Bixa orellana* for preventing and/or reducing sebum secretion and/or for preventing and/or reducing the unaesthetic and/or unpleasant and/or uncomfortable manifestations linked to sebum secretion, wherein the extract of *Bixa orellana* is a seed extract obtained by aqueous extraction.

2. The use as claimed in the preceding claim, wherein the unaesthetic and/or unpleasant and/or uncomfortable manifestations of sebum secretion are skin pore visibility and/or blackhead formation and/or the shiny and/or oily appearance of the skin and/or of the hair and/or the thick appearance of the skin.

3. The use as claimed in the preceding claim, wherein the extract of *Bixa orellana* is spray-dried in the presence of a concentration by weight of maltodextrin of between 20% and 90%, preferentially between 40% and 80%, more preferentially 60%, relative to the total weight of the powder obtained.

4. The use as claimed in one of the preceding claims, wherein the extract of *Bixa orellana* is in the form of a cosmetic composition also comprising a cosmetically acceptable excipient.

5. The use as claimed in claim 4, wherein the cosmetic composition is intended for topical application to the skin, in particular the scalp, and/or the hair.

6. The use as claimed in the preceding claim, wherein the extract of *Bixa orellana* is present in the cosmetic composition in a content of between 1×10⁻⁴% to 10% by weight, preferentially from 1×10⁻⁴% to 5% by weight, more advantageously from 1×10⁻³% to 3% by weight, more preferentially from 0.001% and 0.1% by weight, relative to the total weight of the composition.

7. The use as claimed in either of claims 5 and 6, wherein the skin is normal, oily, very oily and/or mixed and/or the hair is normal and/or oily.

8. The use as claimed in one of claims 5 to 7, wherein the skin is Caucasian or African or Asian and/or the hair is Caucasian, African or Asian.

9. The use as claimed in any one of claims 4 to 8, wherein the cosmetic composition is chosen from the group consisting of a serum, a lotion, a cream, a shampoo, a conditioner, an oil, a milk, an ointment, a paste, a foam, an emulsion, a hydrogel, a shower gel, a mask, a lacquer, a spray, and a wax; it is advantageously a cream, a serum or a lotion.

10. The use as claimed in any one of claims 1 to 9, wherein the extract of *Bixa orellana* is applied to at least one area of the body where the sebum secretion is uncomfortable and/or unaesthetic and/or unpleasant, this area or these areas preferentially being a surface of the body chosen from the skin of the face, including the forehead, the cheeks, the nose, the temples, the T zone (forehead, nose and chin), the external auditory canal and/or the chin, the scalp, the neck, the back, the shoulders, the forearms, the chest, the hands and/or the bust.

11. A water-soluble extract of *Bixa orellana* for use in the treatment and/or prevention of a pathological condition associated with sebum hyperproduction chosen from pathological hyperseborrhoea and seborrhoeic eczema and/or any combination thereof, wherein the extract of *Bixa orellana* is a seed extract obtained by aqueous extraction.

12. The extract of *Bixa orellana* for use as claimed in the preceding claim, wherein the extract is as defined in claim 3.

13. The extract of *Bixa orellana* for use as claimed in either one of claims 11 and 12, wherein the extract is present in the pharmaceutical composition in a content of between 1×10⁻⁴% to 10% by weight, preferentially from 1×10⁻⁴% to 5% by weight, more advantageously from 1×10⁻³% to 3% by weight, more preferentially from 0.001% and 0.1% by weight, relative to the total weight of the composition.
